(19) Europäisches Patentamt
European Patent Office
Office européen des brevets

(11) Numéro de publication: 0 000 678 B1

# (12) FASCICULE DE BREVET EUROPEEN

(45) Date de publication du fascicule de brevet: 05.08.81

(51) Int. Cl.³: C 07 C 101/54, C 07 D 215/44

(21) Numéro de dépôt: 78400059.8

(22) Date de dépôt: 18.07.78

## (54) Nouvel intermédiaire de synthèse: l'anthranilate de glycéryle et son procédé de préparation.

(30) Priorité: 26.07.77 FR 7722899

(43) Date de publication de la demande: 07.02.79 Bulletin 79/3

(45) Mention de la délivrance du brevet: 05.08.81 Bulletin 81/31

(84) Etats Contractants Désignés: BE CH DE GB LU NL SE

(56) Documents cités:
FR - A - 1 421 229
US - A - 3 123 631

(73) Titulaire: PIERRE FABRE S.A.
125, rue de la Faisanderie
F-75116 Paris (FR)

(72) Inventeur: Mouzin, Gilbert, Dr.
21, rue Sainte-Foy
F-81100 Castres (FR)
Inventeur: Cousse, Henri, Dr.
Foun de las Nibios Chemin de Lastinos
F-81100 Castres (FR)

(74) Mandataire: Corre, Jacques Denis Paul et al,
Cabinet Regimbeau 26, Avenue Kléber
F-75116 Paris (FR)

EP 0 000 678 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouvel intermédiaire de synthèse: l'anthranilate de glycéryle et son procédé de préparation

La présente invention, réalisée au Centre de Recherche Pierre FABRE, concerne un nouveau dérivé de l'acide anthranilique et son procédé de préparation.

Plus particulièrement, l'invention concerne un dérivé de formule:

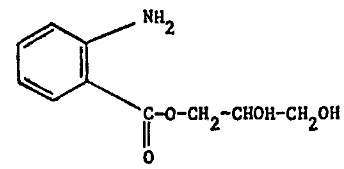

l'anthranilate d'$\alpha$-glycéryle et ses sels, en particulier ses halogénohydrates, qui sont utiles notamment comme intermédiaires de synthèse pour préparer des médicaments.

Le brevet des Etats-Unis d'Amèrique n° 3 123 631 décrit des dérivés d'anthranilate hydroxylés ainsi que des procédés pour leur préparation à partir d'anhydride isatoïque mais aucun composé ayant les caractéristiques du produit selon la présente invention.

Selon la présente invention, ce nouveau dérivé peut être obtenu par condensation du glycérol sur l'anhydride isatoïque selon la schéma réactionnel suivant:

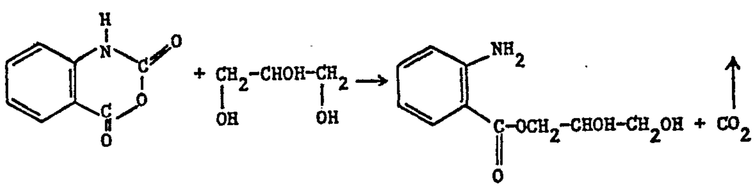

La condensation précédente est mise en oeuvre en présence d'un catalyseur de condensation, en particulier une base, qui peut être minérale, par exemple un hydroxyde de métal alcalin ou alcalino-terreux, ou organique, par exemple la triéthylamine. On utilise, de préférence, comme catalyseur de condensation la soude.

La température et la pression de réaction ne sont pas des paramètres très critiques, néanmoins, compte tenu notamment du dégagement gazeux qui se produit, il est intéressant d'éliminer le gaz formé par pompage en continu, de ne chauffer le mélange réactionnel qu'assez lentement et de maintenir quelque temps, par exemple 1 heure, le mélange réactionnel à une température comprise entre 60 et 90°C.

La réaction est conduite en présence d'un excès molaire de glycérol qui sert de solvant et améliore le rendement de la réaction, cet excès molaire peut être compris, par exemple, entre 2/1 et 20/1 par rapport à l'anhydride isatoïque.

Dans ce procédé il n'est pas nécessaire d'utiliser un glycérol anhydre, car une faible teneur en eau ne modifie pas les rendements de la réaction.

Le procédé selon l'invention permet d'obtenir l'anthranilate d'$\alpha$-glycéryle en solution dans le glycérol qui peut être utilisé tel quel dans d'autres étapes réactionnelles.

Mais, bien entendu, on peut séparer le produit brut par l'un des nombreux procédés connus dans l'industrie chimique. On peut également purifier le produit brut par cristallisation du chlorhydrate.

L'anthranilate d'$\alpha$-glycéryle est un produit intermédiaire dans la synthèse de dérivés de type 4-(2'-carboxyphénylamino)-7-chloroquinoléine ou glafénine qui est un composé antalgique connu.

En effet, par réaction du composé de l'invention sur la 4,7-dichloroquinoléine en milieu chlorhydrique à chaud puis neutralisation, on obtient la glafénine comme cela est décrit dans la demande de brevet déposée le 26 juillet 1977 au nom de la titulaire et ayant pour titre "Nouveau procédé de préparation de la glafénine". (brevet européen n° 0000679).

Ce procédé de préparation de la glafénine présente l'avantage de n'utiliser que des produits largement accessibles dans le commerce et peu coûteux.

Ainsi, l'anhydride isatoïque qui connaît un développement industriel en tant qu'intermédiaire de synthèse dans l'industrie des colorants peut, grâce à de nouveaux procédés de synthèse, être obtenu à des prix très inférieurs aux prix de l'acide anthranilique qui constitue le produit de base dans les synthèses connues de la glafénine.

En outre, ce procédé de synthèse permet d'accroître notablement les rendements en glafénine par rapport aux procédés connus tels que le procédé décrit dans le brevet français 1 421 229 qui utilise comme intermédiaire de synthèse l'acétonide du composé selon la présente invention, notamment en limitant le nombre des étapes de réaction.

A titre d'exemple non limitatif, on décrit, ci-après, la synthèse de composés selon l'invention.

Exemple 1

*Préparation d'anthranilate d'$\alpha$-glycéryle en solution dans le glycérol*

Dans un réacteur de 100 l on introduit 29,3 kg de glycérol (321 moles), on ajoute 157 g de soude en pastilles (3,9 moles) puis 5,1 kg d'anhydride isatoïque (31 moles).

On chauffe lentement, le gaz carbonique qui se dégage est aspiré par l'intermédiaire d'une pompe dont le débit est de 10 $m^3$/heure; le mélange réactionnel est maintenu environ 1 heure entre 60 et 90°C. A ce stade le rendement est quantitatif en anthranilate d'$\alpha$-glycéryle en solution dans le glycérol; ce dérivé peut être utilisé directement pour condenser le groupe amino avec des halogénures d'alcoyles mobiles par exemple.

Exemple 2

*Préparation du chlorhydrate d'anthranilate de glycéryle*

La solution obtenue à l'exemple 1 est traitée pour éliminer le glycérol en excès, le résidu réactionnel est dilué dans l'acétate d'éthyle puis chlorhydraté, soit par barbotage d'acide chlorhydrique gazeux, soit par addition d'éthanol saturé d'acide chlorhydrique.

Le chlorhydrate de l'anthranilate de glycéryle est cristallisé aprés concentration des solvants et addition de méthylal.

On récupère ainsi avec un rendement de 80% environ le composé cristallisé de formule:

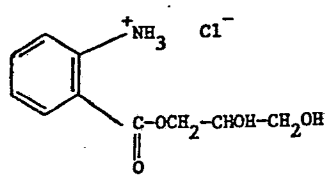

Formule brute: $C_{10}H_{14}ClNO_4$.
Masse moléculaire: 247,6.
Point de fusion: 140°C.
Chromatographie en couche mince:
- support: silice Merck F 254
- solvant: acétate d'éthyle
- révélation: lampe à UV ou vapeurs d'iode
- Rf: 0,41.

Spectrographie infra-rouge:
Appareil Perkin-Elmer modèle 257
Pastille de KBr
$\nu_{C=O}$ (ester) à 1695 $cm^{-1}$
$\nu_{OH}$ à 3400 $cm^{-1}$
Bandes de salification 2500 à 2900 $cm^{-1}$.

*Caractéristiques de l'anthranilate de glycéryle.*
Formule:

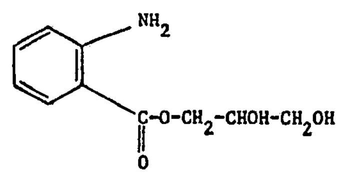

Produit huileux, légèrement jaunâtre.
Spectre infra-rouge effectué entre lames de NaCl:
$\nu_{OH}$ et $\nu_{NH}$ entre 3300 et 3500 $cm^{-1}$
$\nu_{CH}$ (aromatiques) à 3040—3060 et 3080 $cm^{-1}$
$\nu_{C=O}$ centrée à 1700 $cm^{-1}$
$\nu_{C-C}$ aromatiques à 1590 et 1620 $cm^{-1}$.

**Revendications**

1. Un composé de formule:

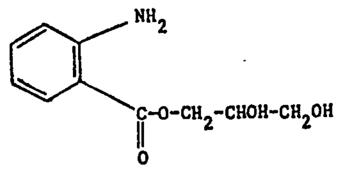

(I)

et ses sels.

2. Le composé de formule I sous forme de chorhydrate.

3. Procédé de préparation d'un composé de formule:

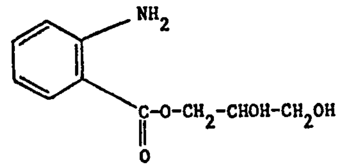

en solution dans le glycérol, caractérisé en ce que l'on condense le glycérol sur l'anhydride isatoïque de formule:

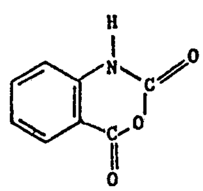

en présence d'un catalyseur de condensation en utilisant un excès molaire du glycérol par rapport à l'anhydride isatoïque.

4. Procédé selon la revendication 3, caractérisé en ce que le catalyseur de condensation est une base minérale ou organique.

5. Procédé selon la revendication 4, caractérisé en ce que le catalyseur de condensation est un hydroxyde de métal alcalin ou alcalino-terreux.

6. Procédé selon la revendication 5, caractérisé en ce que le catalyseur de condensation est la soude.

7. Procédé selon l'une des revendications 3 à 6, caractérisé en ce que l'excès molaire de glycérol par rapport à l'anhydride isatoïque est compris entre 2/1 et 20/1.

8. Application du composé selon l'une des revendications 1 et 2 comme intermédiaire dans la synthèse de la glafénine par action de ce composé sur la 4,7-dichloroquinoléine.

**Claims**

1. A compound of the following formula:

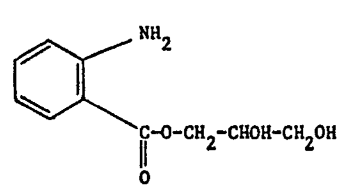

(I)

and the salts thereof.

2. The compound of formula I in the form of hydrochloride.

3. A process for the preparation of a compound of the following formula:

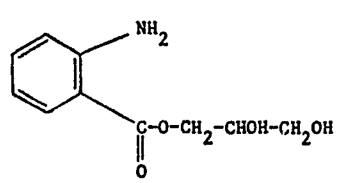

in solution in glycerol, characterised in that glycerol is condensed with isatoic anhydride of the following formula:

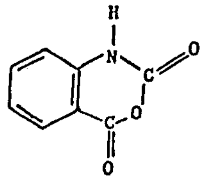

in the presence of a condensation catalyst using a molar excess of glycerol relative to the isatoic anhydride.

4. A process according to claim 3, characterised in that the condensation catalyst is an inorganic or organic base.

5. A process according to claim 4, characterised in that the condensation catalyst is an alkali metal hydroxide or an alkaline-earth metal hydroxide.

6. A process according to claim 5, characterised in that the condensation catalyst is soda.

7. A process according to one of claims 3 to 6, characterised in that the molar excess of glycerol relative to the isatoic anhydride is from 2/1 to 20/1.

8. The use of the compound according to one of claims 1 and 2 as an intermediate in the synthesis of glafenine by the action of this compound on 4,7-dichloroquinoline.

**Patentansprüche**

1. Verbindung der Formel

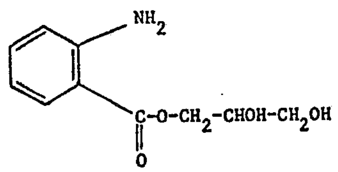

(I)

und deren Salze.

2. Verbindung nach Anspruch 1 in Form des Chlorhydrats.

3. Verfahren zur Herstellung einer Verbindung gemäß Formel

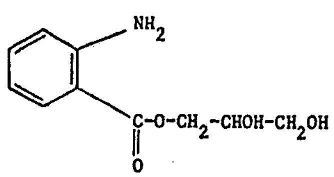

in lösung in Glyzerin, dadurch gekennzeichnet, daß man das Glyzerin mit Isatoïcsäureanhydrid gemäß Formel

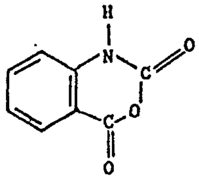

in Gegenwart eines Kondensationskatalysators kondensiert, wobei man einen molaren Überschuß Glyzerin, bezogen auf das Isatoïcsäureanhydrid, verwendet.

4. Verfahren nach Anspruch 3, dadurch gekennzeichnet, daß man als Kondensationskatalysator eine organische oder eine Mineralbase verwendet.

5. Verfahren nach Anspruch 4, dadurch gekennzeichnet, daß man als Kondensationskatalysator ein Alkalimetall- oder Erdalkalimetallhydroxid verwendet.

6. Verfahren nach Anspruch 5, dadurch gekennzeichnet, daß man als Kondensationskatalysator Natriumkarbonat (Soda) verwendet.

7. Verfahren nach einem der Ansprüche 3 bis 6, dadurch gekennzeichnet, daß man das Glyzerin, bezogen auf das Isatinsäureanhydrid, in molarem Überschuß von 2:1 bis 20:1 verwendet.

8. Verwendung der Verbindung nach einem der Ansprüche 12 und/oder 2, als Zwischenprodukt bei der Synthese von Glafeninum durch Umsetzung dieser Verbindung mit 4,7-Dichlorchinolin.